Europäisches Patentamt

European Patent Office

Office européen des brevets

(11) Veröffentlichungsnummer: **0 051 729**
**A1**

(12) **EUROPÄISCHE PATENTANMELDUNG**

(21) Anmeldenummer: 81107439.2

(51) Int. Cl.³: **A 61 F 1/03**

(22) Anmeldetag: 19.09.81

(30) Priorität: **10.11.80 DE 3042385**

(43) Veröffentlichungstag der Anmeldung: **19.05.82**
**Patentblatt 82/20**

(84) Benannte Vertragsstaaten: **CH FR GB LI SE**

(71) Anmelder: **Waldemar Link GmbH & Co,**
**Barkhausenweg 10, D-2000 Hamburg 63 (DE)**

(72) Erfinder: **Keller, Arnold, An der Naher Furth 5,**
**D-2061 Kaihude (DE)**

(74) Vertreter: **Glawe, Richard, Dr. Dipl.-Ing. et al, Glawe,**
**Delfs, Moll & Partner Rothenbaumchaussee 58,**
**D-2000 Hamburg 13 (DE)**

(54) Alloplastischer Gelenkflächenersatz.

(57) In einer Gelenkprothese erstreckt sich der alloplastische Gelenkflächenersatz (2, 4) nur über einen Teil der gesamten Gelenkfläche. Wenn beide Gelenkteile alloplastisch ersetzt werden sollen, sind die aufeinander zu gleiten bestimmten Implantate (2, 4) so geformt, daß sie einander in allen Stellungen des Gelenks, in denen Lastbewegung stattfinden kann, zumindest teilweise überdecken.

0051729

— / —

Beschreibung

Die Erfindung bezieht sich auf einen alloplastischen Gelenkflächenersatz.

Bekannte Gelenkprothesen oder -implantate ersetzen jeweils die gesamte Gelenkfläche. Unter der Gelenkfläche ist in diesem Zusammenhang die gesamte, von Knorpelgewebe gebildete Oberfläche eines Gelenks zu verstehen, die ständig oder in bestimmten Gelenkstellungen gleitend mit einer Gelenkgegenfläche zusammenwirkt. Beispielsweise wird bei Hüftgelenksprothesen der Hüftkopf entweder gänzlich durch eine Alloplastik ersetzt oder mit einer Kappenprothese abgedeckt, nachdem seine Knorpelschicht zuvor abgefräst wurde. Die Gelenkpfanne wird in der Regel ausgefräst und in ihrer Gesamtheit mit einer Pfannenprothese ausgefüttert. Eine solche Operation kann Erleichterung bringen; jedoch verursachen biologische Reaktionen des die Implantate umgebenden Gewebes im Laufe der Zeit nicht selten Mißerfolge. Je ausgedehnter das natürliche Gewebe durch eine Alloplastik ersetzt wurde, um so schwerer ist es, durch spätere Eingriffe wieder befriedigende Verhältnisse herzustellen.

Der Erfindung liegt daher die Aufgabe zugrunde, einen alloplastischen Gelenkflächenersatz zu schaffen, der mit geringstmöglicher Resektion auskommt.

0051729

— 2 —

Die erfindungsgemäße Lösung besteht darin, daß das als Gelenkflächenersatz vorgesehene Implantat sich nur über einen Teil der Gelenkfläche erstreckt, und zwar vorzugsweise über denjenigen Gelenkflächenteil, der im gesunden Gelenk durch Struktur und Knorpeldicke als hauptsächlich tragend erkennbar ist. Jedes Gelenk zeigt durch die Struktur des die Gelenkfläche bildenden Knorpels und des darunter liegenden Knochengewebes sowie durch die Dicke des Knorpels deutlich die Zonen an, die der Belastung hauptsächlich ausgesetzt sind. Häufig sind diese Zonen durch eine deutliche Grenzlinie von der übrigen, nicht oder wenig tragenden Gelenkfläche unterschieden. Beispielsweise ist die Hüftpfanne bei einem Menschen halbkugelig ausgebildet, wobei der sie auskleidende Knorpel in seiner Gesamtheit die Gelenkfläche bildet. Der die Hauptbelastungszone bildende Knorpel überzieht den Hüftpfannenboden jedoch nur in der Form eines hufeisenförmigen Streifens. Im Hauptbelastungsbereich ist dieser Knorpelstreifen gemäß der natürlichen stärksten Belastung am dicksten und nimmt zu den beiden Enden des Streifens an Stärke ab. Die Erfindung sieht in diesem Beispiel nur den Ersatz des hufeisenförmigen Knorpelstreifens in der Hüftpfanne vor. Entsprechend kann das Implantat am Hüftkopf beschränkt werden auf einen etwa ovalen Streifen, der den Kopf auf einem Drittel seines Umfangs oder mehr umgibt und dessen Breite nur ungefähr die Hälfte der entsprechenden Dimension der Hüftkopfgleitfläche beträgt.

Während in diesem Beispiel beide zusammenwirkenden Implantate auf einen Teil der Gleitfläche begrenzt ist, ist es im Rahmen der Erfindung selbstverständlich möglich,

- 5 -

eines der beiden Implantate in herkömmlicher Weise über
die gesamte Gleitfläche zu erstrecken. Ferner gelten
für die Materialwahl die bekannten Grundsätze, also
beispielsweise die Paarung von Implantaten aus hartem,
glatten Material (Metall, Keramik) einerseits und Kunstharz (insbesondere Polyethylen) andererseits. Aber auch
andere Werkstoffe sind verwendbar.

In vielen Fällen wird es zweckmäßig sein, ein Implantat
oder beide zusammenwirkenden Implantate schwach aus der
Fläche des nicht ersetzten Knorpelgewebes herauszuheben,
wie dies auch bei dem gesunden Gelenkknorpel im Hauptbelastungsbereich der Fall ist. Um die Bildung von Kanten
am Übergang vom Implantat zu der verbleibenden, natürlichen
Gelenkfläche zu vermeiden, werden die Ränder des Implantats
zweckmäßigerweise abgerundet, abgeschrägt oder in sonstiger
Weise auf die Höhe der verbleibenden Gleitfläche zurückgeführt. Ferner ist es vorteilhaft, die Form von zwei aufeinander zu gleiten bestimmten Implantaten so aufeinander abzustimmen, daß sie außerhalb der Hauptbelastungsstellungen
zumindest in denjenigen Stellungen, in denen eine Lastbewegung mitunter vorkommen kann, einander noch zumindest
teilweise überdecken. Besser ist es, wenn eine gewisse
Mindestüberdeckung in sämtlichen in Frage kommenden
(auch nicht belasteten) Stellungen erhalten bleibt. Dadurch
soll vermieden werden, daß die Kante eines Implantats unter
Last gegen die des anderen schlägt.

Aus dem Bestreben, so wenig natürliches Gewebe als nur
möglich alloplastisch zu ersetzen, folgt als vorteilhafte
Ausführungsform des erfindungsgemäßen Implantats eine
möglichst flache Formgebung, deren Dicke lediglich durch

0051729

— 4 —

die Höhe des zu ersetzenden Gewebes, durch die notwendige Formbeständigkeit des Implantats und die Notwendigkeit einer Verankerung vorgegeben ist. Für die Verankerung werden beispielsweise Stifte oder eine geeignete unterseitige Strukturierung vorgesehen.

Entsprechend den unterschiedlichen Radien der in der Praxis vorkommenden Gelenke müssen die Implantate in entsprechend unterschiedlichen Radien zur Verfügung stehen.

Durch den Einsatz der erfindungsgemäßen Implantate entfällt gegenüber dem Stand der Technik die Notwendigkeit, die gesamten Gelenkflächen passend zu bearbeiten. Dadurch ergibt sich die weitgehende Erhaltung des natürlichen Knochens als tragendes Element. Nur die zerstörten Belastungsfelder werden je durch ein Implantat gleichförmig ersetzt. Somit ist die Ernährung des Knochens gegenüber anderen Methoden besser zu erhalten. Evtl. notwendige spätere Behandlungen sind mit geringerem Aufwand und Risiko verbunden.

Die Erfindung wird im folgenden näher unter Bezugnahme auf das in der Zeichnung dargestellte Beispiel erläutert. Darin zeigen:

Fig. 1          den Hüftknochen, gesehen auf die Hüftpfanne, und

Fig. 2 und 3      Schnittansichten des Hüftgelenks mit zwei unterschiedlichen Hüftkopfimplantaten.

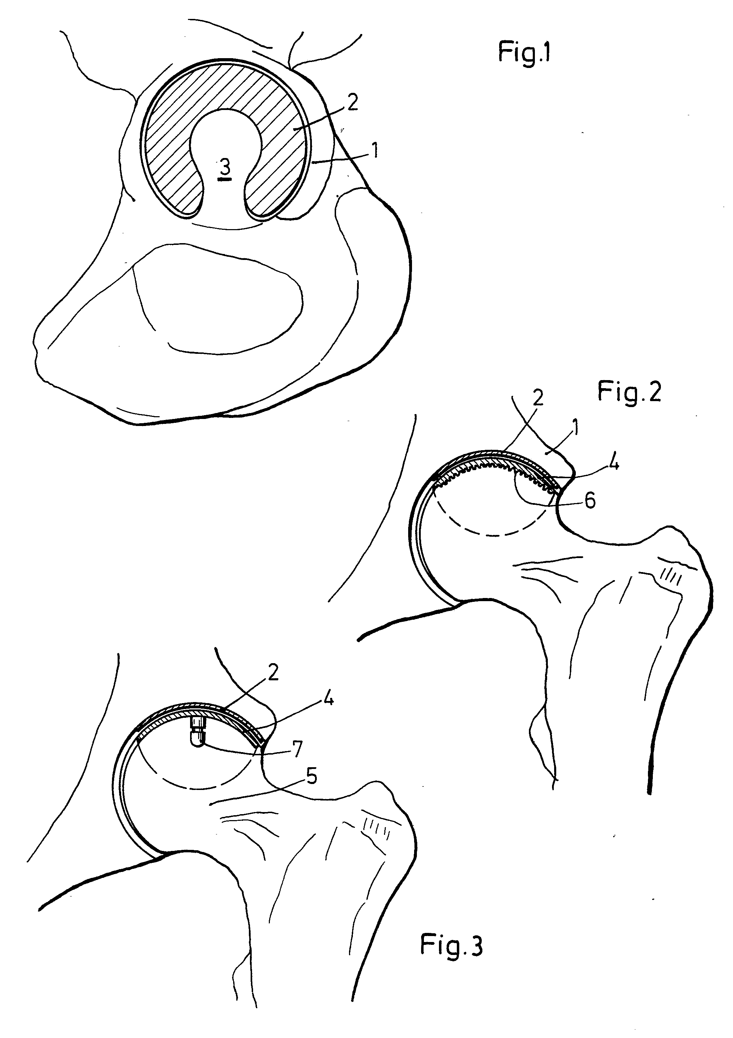

Man erkennt in Fig. 1, daß die Hüftpfanne 1 ein Implantat 2 von hufeisenförmiger Gestalt enthält. Der

— 5 —

Bereich 3 der Gelenkfläche bleibt mit seinem natürlichen Gewebe erhalten. In den Figuren 2 und 3 erkennt man das Implantat 2 im Schnitt. Ihm steht ein Implantat 4 am Hüftkopf 5 gegenüber, das einen Teil von dessen die Gelenkfläche bildenden Knorpelschicht ersetzt und im Falle der Fig. 2 durch rückseitige Strukturierung, die mit dem Knochengewebe verwächst oder durch Knochenzement daran verankert ist, und im Beispiel der Fig. 3 durch einen Verankerungsstift 7 gehalten ist.

GLAWE, DELFS, MOLL & PARTNER

PATENTANWÄLTE
ZUGELASSENE VERTRETER BEIM EUROPÄISCHEN PATENTAMT

RICHARD GLAWE
DR - ING

KLAUS DELFS
DIPL - ING

ULRICH MENGDEHL
DIPL - CHEM. DR RER NAT

WALTER MOLL
DIPL - PHYS DR. RER NAT.
ÖFF. BEST DOLMETSCHER

HEINRICH NIEBUHR
DIPL - PHYS DR PHIL HABIL

8000 MÜNCHEN 26
POSTFACH 37
LIEBHERRSTR. 20
TEL. (089, 22 65 48
TELEX 52 25 05 SPEZ

2000 HAMBURG 13
POSTFACH 25 70
ROTHENBAUM-
CHAUSSEE 58
TEL. (040) 4 10 20 08
TELEX 21 29 21 SPEZ

HAMBURG

0051729

Alloplastischer
Gelenkflächenersatz

_____

Waldemar Link GmbH & Co
Hamburg

_____

p 10149/81 EU
D/be

## Patentansprüche

1. Alloplastischer Gelenkflächenersatz, dadurch gekennzeichnet, daß er sich nur über einen Teil der Gelenkfläche erstreckt.

2. Gelenkflächenersatz nach Anspruch 1, dadurch gekennzeichnet, daß er im wesentlichen denjenigen Gelenkflächenteil ersetzt, der im gesunden Gelenk durch
   Struktur und Knorpeldicke als hauptsächlich tragend
   erkennbar ist.

3. Gelenkflächenersatz nach Anspruch 1 oder 2, dadurch
   gekennzeichnet, das zumindest seine den Übergang zu
   natürlicher Gelenkfläche bildenden Ränder abgeflacht oder
   abgerundet sind.

0051729

4. Gelenkflächenersatz nach einem der Ansprüche 1 bis 3, dadurch gekennzeichnet, daß ein Paar von aufeinander zu gleiten bestimmten Implantaten (2, 4) so geformt ist, daß sie außerhalb der Hauptbelastungsstellungen zumindest in denjenigen Stellungen, in denen Lastbewegung auch noch möglich ist, einander teilweise überdecken.

5. Gelenkflächenerstaz nach einem der Ansprüche 1 bis 4, dadurch gekennzeichnet, daß das Hüftpfannenimplantat (2) hufeisenförmig ist.

6. Gelenkflächenimplantat nach einem der Ansprüche 1 bis 5, dadurch gekennzeichnet, daß es als Hüftkopfimplantat (4) ovale Streifenform hat und sich um mindestens ein Drittel des Hüftkopfumfangs erstreckt.

1/1

0051729

Fig.1

Fig.2

Fig.3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | <u>DE - A - 2 933 174</u> (FREY)<br>* Figuren 1,5,6; Ansprüche 1,2; Seite 1, Zeilen 3-6; Seite 4, Zeilen 31-34; Seite 5, Zeilen 1-5 *<br>-- | 1-3,5 |
| X | <u>DE - A - 1 964 781</u> (LINK)<br>* Zeichnung; Seite 2, Zeilen 19-23 *<br>-- | 1,2 |
| X | <u>DE - A - 2 102 454</u> (MORGAN)<br>* Zeichnung; Anspruch 1; Seite 2, letzter Absatz; Seite 3; Seite 4, Zeilen 1-4, 8-11 *<br>-- | 1,2 |
| X | <u>FR - A - 2 242 068</u> (OSCOBAL A.G.)<br>* Figuren 3,4; Seite 1, Zeilen 10-32; Seite 3, Zeilen 23-25 *<br>& DE - A - 2 411 618<br>-- | 1,2 |
| X | <u>FR - A - 2 229 382</u> (CHARNLEY)<br>* Seite 1, Zeilen 36-39; Seite 2, Zeilen 1-13 *<br>& DE - A - 2 421 045<br>-- | 1 |
| X | <u>EP - A - 0 017 930</u> (PRUSSNER et al.)<br>* Figur 1; Seite 1, Zeilen 21-23; Seite 2, Zeilen 1-6; Seite 3, Zeilen 7-33; Seite 4, Zeilen 1-4 *<br>./.<br>-- | 1,3 |

**KLASSIFIKATION DER ANMELDUNG (Int. Cl.³)**

A 61 F 1/03

**RECHERCHIERTE SACHGEBIETE (Int. Cl.³)**

A 61 F

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 05-02-1982 | FISCHER |

Europäisches
Patentamt

**EUROPÄISCHER RECHERCHENBERICHT**

0051729

Nummer der Anmeldung

EP 81 10 7439

| | **EINSCHLÄGIGE DOKUMENTE** | | KLASSIFIKATION DER ANMELDUNG (Int.Cl.) |
|---|---|---|---|
| Kategorie | Kennzeichnung des Dokuments mit Angabe, soweit erforderlich, der maßgeblichen Teile | betrifft Anspruch | |
| A | US - A - 2 910 978 (URIST)<br><br>* Figuren 1-4; Spalte 2, Zeilen 53-60; Spalte 4, Zeilen 47-51 *<br><br>---- | 1,5 | RECHERCHIERTE SACHGEBIETE (Int. Cl.) |